# EUROPEAN PATENT SPECIFICATION

(11) **EP 1 054 258 B1**
(45) Date of publication and mention of the grant of the patent: **07.12.2005**
(21) Application number: 00109057.0
(22) Date of filing: 28.04.2000
(51) Int. Cl.: G01N 33/48, G01N 33/543, G01N 33/58

(54) **Homogeneous enzyme immunoassay process using second antibody**
Homogenes Enzym-Immunoassay- Verfahren mit zweitem Antikörper
Méthode homogène de dosage immunologique enzymatique faisant intervenir un second anticorps

(30) Priority: 28.04.1999 JP 12094099
(43) Date of publication of application: 22.11.2000
(73) Proprietor: FUJI PHOTO FILM CO., LTD., Kanagawa 250-01 (JP)
(72) Inventor: Shinoki, Hiroshi, Asaka-shi, Saitama (JP); Seshimoto, Osamu, Asaka-shi, Saitama (JP)
(74) Representative: Albrecht, Thomas

(56) References cited:
- EP-A- 0 182 546
- EP-A- 0 579 250
- EP-A- 0 813 064
- US-A- 4 203 802
- US-A- 4 235 960
- US-A- 4 530 900

## Description

### FIELD OF THE INVENTION

The present invention relates to an immunoassay for quantitatively determining a trace constituent present in a sample, in which a reaction between an antigen and an antibody is utilized. More particularly, the present invention relates to a homogeneous enzyme immunoassay for quantitatively determining an analyte antigen (ligand) in the sample by the use of an enzyme-labeled antigen.

### BACKGROUND OF THE INVENTION

Analyses of the constituents originated from the living body or chemicals contained in the body fluids, such as blood or urine, are useful for diagnosing the condition of diseases or judging the course of curing, and thus they occupy important parts in the field of clinical test. The so-called enzyme immunoassay has been known in the art as one method for analyzing such constituents (ligands) generally present in a small amount in the body fluids. The enzyme immunoassay may be classified into a heterogeneous system for which B/F (Bound/Free) separation must be effected, and a homogeneous system in which B/F (Bound/Free) separation is not necessary. Meantime, B stands for the labeling material in a complex formed by binding of the specific antibody (or specific antigen) to the ligand, and F stands for the free labeling material which is not bound to the ligand.

In the heterogeneous system, the antigen-antibody bound (B) formed by the reaction between the antigen and the antibody is separated from free antibody and antigen (F) by any suitable means and then the activity of the labeling enzyme in the antigen-antibody bound is determined. Although it is expected that the heterogeneous system has a high sensitivity in principle since the bound (B) is separated from free antibody and antigen (F), there is a problem that cumbersome operations are needed for the B/F separation and thus a relatively long time is necessary for the determination.

On the other hand, the reaction in the homogeneous system are based on the phenomenon that the enzymatic activity of the labeling enzyme is affected by some interference caused by binding of an antibody to the antigen (ligand), and the inhibition due to antigen-antibody binding is generally utilized. In general, the antigen is labeled with an enzyme so that the suppression in enzymatic activity either by a steric hindrance imposed on binding of the enzyme, which is bound to a generally large molecule antibody, with the substrate or by a change in three-dimensional structure of the enzyme is detected. For example, EMIT (Enzyme Multiplied Immunoassay Technique) is well known as such a system. The ligand will compete with the enzyme-labeled ligand in the binding to the antibody. When a large amount of the ligand exists, free enzyme-labeled ligand unbound with the antibody remains in a large amount, and thus the enzymatic activity of the labeling enzyme is maintained. When the amount of the ligand is small, the enzyme-labeled ligand binds to the antibody, then the enzymatic activity decreases. Therefore, the enzymatic activity of the labeling enzyme increases in proportion to the quantity of the antigen (ligand) in the sample.

The operations in the homogeneous system are thus relatively simplified since complicated B/F separation is not necessary. However, the suppression in enzymatic activity of the labeling enzyme caused by binding of the antibody is not always sufficient. In such a case, the enzymatic activity value (background value) is not low enough when no antigen (ligand) is present, and thus, a sufficient S/N ratio cannot be ensured.

On the other hand, in order to enhance the sensitivity for detecting an antigen in a sample, i.e., to lower the limit for detecting the antigen, it is enough to reduce the quantity of the antibody. However, if the quantity of the antibody is reduced, the quantity of the antibody bound to the enzyme-labeled antigen also decreases and, as a result, the inhibitory effect against the labeling enzyme decreases. This result means increase of the background value, leading to a reduction of the S/N ratio.

### OBJECTS AND SUMMARY OF THE INVENTION

The present invention has been accomplished in view of the aforementioned circumstances, and an object thereof is to provide a homogeneous enzyme immunoassay process for enabling to ensure a sufficient S/N ratio with a low background value. Furthermore, another object of the present invention is to provide a homogeneous enzyme immunoassay process for enabling to obtain a S/N ratio not lower rather higher than that observed before reducing the quantity of an antibody even when the quantity of an antibody relative to an antigen is reduced in order to enhance the sensitivity.

The objects of the present invention are attained by the provision of a homogeneous enzyme immunoassay process for quantitatively analyzing a ligand, comprising of: reacting the ligand with an enzyme-labeled ligand, an antibody against the ligand, and a second antibody against said antibody, and then determining a change in enzymatic activity of a labeling enzyme of said enzyme-labeled ligand in the reaction mixture.

In the present invention, steric hindrance is enhanced by binding the enzyme-labeled ligand (antigen) to the antibody, followed by successive binding to the second antibody.

The enzymatic activity of the labeling enzyme in the reaction mixture can be determined by adding a substrate to the reaction mixture. If necessary, it is possible to add a color-developing reagent composition to the enzymatic reaction product, thereby causing color development in proportion to the enzymatic activity and then to carry out colorimetric analysis.

A water-insoluble substrate can be used as the enzyme substrate. Alternatively, the enzymatic activity may be determined by a dry analysis element equipped with a substrate layer containing a water-insoluble substrate. The water-insoluble substrate is generally a macromolecule having a large molecular weight. When a high molecular weight substance has a bonded structure (cross-linked structure) between some of the high polymer chains, it is impossible to separate them. The water-insoluble substrate therefore swells in a solvent but is not soluble therein. Such an insoluble substrate reacts with an enzyme on the surface of its particles. Namely, the enzymatic reaction takes place at the solid-liquid interface, which increases the influence of the steric hindrance against the enzyme. In the present invention, with a view to allowing the steric hindrance to exhibit more easily, an antibody is reacted with an enzyme-labeled antigen, the reaction mixture is reacted with and bound to a second antibody and then the enzymatic activity is preferably measured using an insoluble substrate.

### BRIEF DESCRIPTION OF THE DRAWINGS

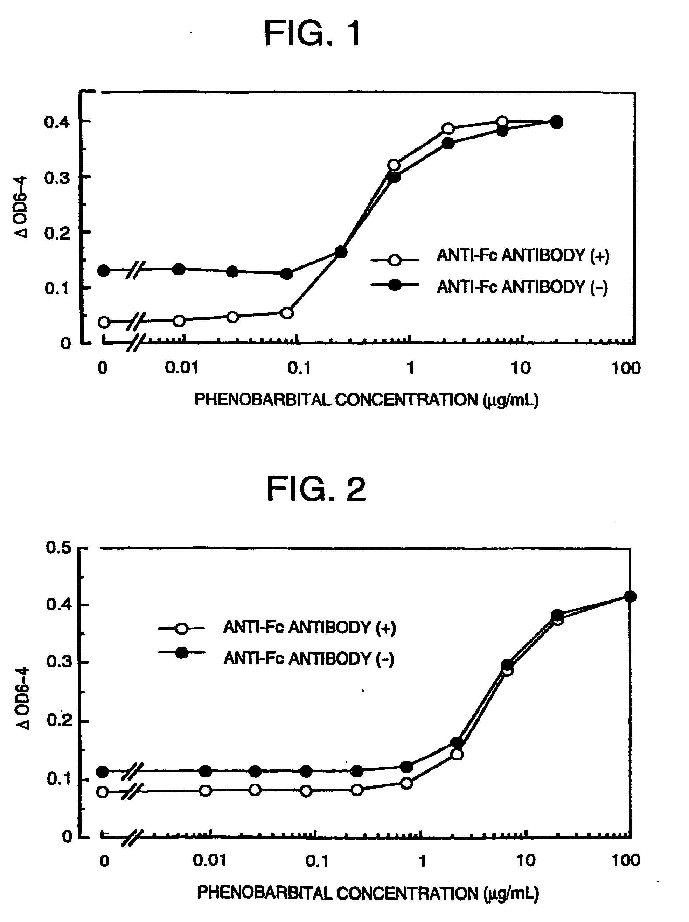

FIG. 1 illustrates calibration curves drawn as a result of the analysis in Example 6. In the drawing, the symbol -●- indicates the case where an enzyme-labeled antigen and an antibody were reacted with an antigen (the Comparative Example), while the symbol -○- indicates the case where the enzyme-labeled antigen, first antibody and second antibody were reacted with an antigen in accordance with the present invention.
FIG. 2 illustrates calibration curves drawn as a result of the analysis in Example 8. In the drawing, the symbol -●- indicates the case where an enzyme-labeled antigen and an antibody were reacted with an antigen (the Comparative Example), while the symbol -○- indicates the case where the enzyme-labeled antigen, first antibody and second antibody were reacted with an antigen in accordance with the present invention.

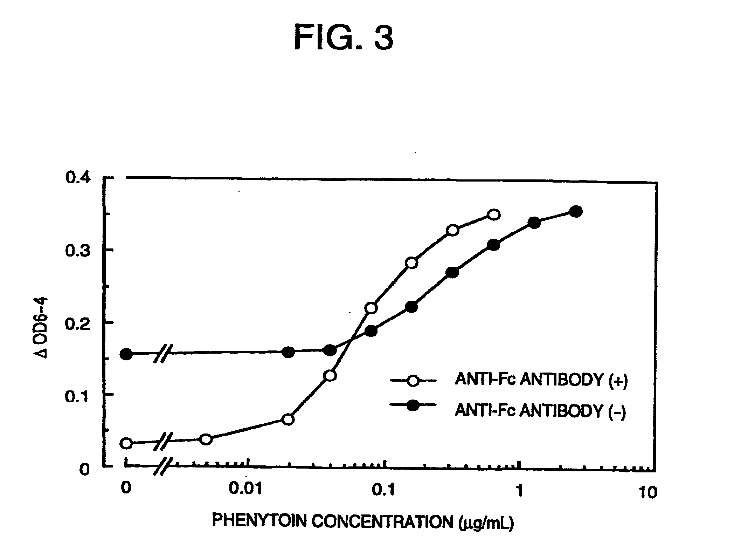

FIG. 3 illustrates calibration curves drawn as a result of the analysis in Example 12. In the drawing, the symbol -●- indicates the case where an enzyme-labeled antigen and an antibody were reacted with an antigen (the Comparative Example), while the symbol -○- indicates the case where the enzyme-labeled antigen, first antibody (one half of the quantity in the Comparative Example) and second antibody were reacted with an antigen in accordance with the present invention.

### DESCRIPTION OF PREFERRED EMBODIMENTS

### Analyte (Substance to Be Analyzed)

The substance to be analyzed by the present invention (hereinafter also referred to simply as "analyte") is a ligand having an antigenic determinant and contained in the sample, that is, an antigen. The sample containing the analyte is not limited and many kinds of sample may be analyzed by this invention. Typical examples include blood (whole blood, blood plasma, blood serum), lymph fluid and urine.

Any ligand including from a low molecular weight substance to a high molecular weight substance, can be analyzed by the use of the analysis element according to the present invention, insofar as each ligand has antigenicity and an antibody thereagainst can be available. Incidently, the expression "the ligand has antigenicity" means that it is capable of reacting with a corresponding antibody, and the ligand is only required to possess an epitope (antigenic determinant). Even a substance having no immunogenecity in itself can serve as a ligand (antigen) to be analyzed in the present invention insofar as it is capable of producing a corresponding antibody by immunizing as a hapten.

In the case that the ligand to be analyzed is an antigen having a high molecular weight which reveals an interference or inhibition to the enzymatic activity when labeled with an enzyme, it suffices to select other low molecular weight substance having an antigenic determinant common to that of the ligand and use the conjugate of such substance and the labeling enzyme as an enzyme-labeled antigen, as will be described specifically hereinafter.

The term "enzyme-labeled ligand" as usedherein conceptually includes not only the one obtained by labeling a ligand (analyte antigen) with an enzyme but also the one obtained by labeling a substance having on antigenic determinant common to that of the ligand with an enzyme. Not only such compounds as ligand derivatives which are analogues in terms of chemical structure but also such compound exhibiting a behavior similar to the ligand in terms of the immunoresponsibility to the antibody can be labeled with an enzyme and used as the enzyme-labeled ligand.

### Enzyme-Labeled Ligand

The enzyme-labeled ligand is a linked product of an analyte ligand (or a ligand-like substance of which antigenic determinant is common to that of the ligand) with an enzyme. When the ligand is a substance having a low molecular weight such as chemicals, it may be directly linked with an enzyme. In the case that the ligand is a substance having a high molecular weight such as a protein, which interferes the enzymatic activity when it is directly bound in its unmodified form with an enzyme, the protein may be fragmented, and a fragment thereof may be used as a substance to be labeled with an enzyme. Sole requirement is that the antigenic determinant of the fragmented protein having a lower molecular weight is common to that the intact protein i.e., the ligand.

The method for linking the ligand with an enzyme can be selected in consideration of the functional groups of both substances. Utilizable functional groups include, for example, amino, carboxyl, hydroxyl, thiol, imidazole, and phenyl. For example, amino groups may be linked to each other by a number of known methods such as the isocyanate method, the glutaraldehyde method, the difluorobenzene method, and the benzoquinone method. An amino group may be linked to a carboxyl group by a method in which a carboxyl group is converted to succinimide ester, or by other methods including the carbodiimide method and the Woodward reagent method. The periodic acid oxidation method (Nakane method) by which an amino group is cross-linked with a sugar chain is also applicable. When a thiol group is utilized, one of carboxyl groups is converted to succinimide ester and then cysteine is reacted therewith to introduce a thiol group, followed by linking both groups by using a bifuctional linking reagent which reacts with the thiol group. The method in which the phenyl group is utilized includes the diazotization method and the alkylation method.

The linking method is not limited to the aforementioned methods, and may be selected from the methods described in "Method in Immunology and Immunochemistry", Vol. 1, (C.A. Williams, M.W. Chase, Academic Press (1967)) or "KOHSO MEN'EKI SOKUTEI-HO (Enzyme Immunoassay)", edited by Ishikawa, Kawai and Miyai, published by Igaku Shoin, 1978. The linking ratio between the ligand and the enzyme is not limited to 1:1, but may be changed to a desired ratio in consideration of the applied use of the product. After the completion of the linking reaction, the reaction product is refined by the gel filtration or the ion exchange chromatography, and may be dried by the lyophilizing process as desired.

In lieu of the ligand, the enzyme may be linked to a derivative of the ligand having immunological cross-reactivity to a corresponding antibody for the ligand. Meanwhile, the derivatives of the ligand include not only those which have analogous chemical structures but also those which exhibit analogous behaviors in their immunological reactivities. For instance, when an antibody against theophylline as the ligand cross-reacts immunologically with caffeine, a derivative of caffeine may be used as a material for the enzyme-labeled ligand.

When the ligand or a derivative thereof has not a proper functional group to be linked to the enzyme, an amino group, a carboxyl group or a thiol group may be introduced into the ligand or the derivative thereof. Such a group may be introduced through a spacer to facilitate linking thereof to the enzyme. For example, when the ligand is theophylline, the carboxyl group may be introduced to obtain 8-propylcarboxyl-theophylline which is linked to the enzyme.

### Antibody

A specific antibody against the ligand to be analyzed is used as the antibody. When a derivative of the ligand is used for preparing the enzyme-labeled ligand, an antibody which reacts with the antigenic determinant common to the ligand and the derivative thereof is used. The antibody may be a polyclonal antibody obtained by the conventional process, a monoclonal antibody may be preferably used to improve the sensitivity. Moreover, the antibody may be a fragment of F(ab')₂, Fab', Fab. However, it is not particularly necessary to use such a fragment. Rather, it is advantageous to use the intact antibody (IgG) which is not such a fragmented antibody in order to increase steric hindrance toward the enzyme-labeled ligand. In addition, since IgG contains Fc region, the use of IgG as the first antibody is rather preferable, which make it possible to employ an anti-Fc antibody as the second antibody.

### Second Antibody

The second antibody is an antibody which recognizes and binds to the first antibody, and may be a monoclonal antibody or a polyclonal antibody. When the first antibody is obtained from a mouse, an anti-mouse IgG antibody may be used as the second antibody. Such the second antibody includes a polyclonal antibody obtained by immunizing an animal (goat, sheep, rabbit or the like) other than mouse with the first antibody. When the first antibody is an intact mouse IgG, a commercially available anti-mouse Fc antibody may be used as the second antibody.

### Labeling Enzyme

The enzyme for forming the enzyme-ligand complex can be selected in consideration of the combination with the substrate which is used at the subsequent enzymatic reaction and a detection system of the enzymatic reaction product. In the present invention, since the reactivity of the enzyme with the substrate is suppressed by the steric hindrance resulted from the formation of an enzyme-antigen-antibody-second antibody complex, it is preferred to select a combination of the enzyme and the substrate from which influence by the steric hindrance is easily detected.

In short, a substrate having a relatively high molecular weight is preferred for attaining a higher sensitivity. For example, a substrate having a molecular weight of not less than about 20,000, preferably not less than about 100,000 is used in the invention. Examples of such substrates include a starch as the substrate for an enzyme amylase; a cellulose as the substrate for an enzyme cellulase; proteins such as gelatin and hemocyanin as the substrate for protease; and various oils and fats as the substrate for lipase. Detailed reports relating to the selection of the enzymes and substrates are disclosed in Unexamined Japanese Patent Publication Nos. 108756/1985 (corresponding to USP 4,692,404 and EP 0144176A), 171461/1985 (corresponding USP 4,757,001 and EP 0152305A) and 171460/1985 (corresponding USP 4,757,001 and EP 0152305A). Amylase used with starch as the substrate is preferred among them. It is particularly preferred that the substrate is a water-insoluble substrate, since the steric hindrance by the presence of the enzyme-antibody-antigen matrix structure is noticeably appeared. Examples of such an enzyme include a hydrolase which forms a diffusible oligomer from a non-diffusible (water insoluble) substrate composed of polymers, a specific example being a glucosidase. Examples of glucosidases include endo-active glucosidases such as α-amylase, β-amylase and dextranase.

### Water-Insoluble Substrate

When an α-amylase is used as the labeling enzyme, carboxymethylated starch, starch, amylose, amylopectin or the like may be used as the substrate. It is particularly preferred for the improvement in sensitivity to use water-insoluble starch, since the enzymatic reaction takes place on the surfaces of the substrate particles, namely the reaction takes place at the solid-liquid interface to exaggerate the influence of steric hindrance to the enzymatic activity by the occurrence of antigen-antibody binding. Alternatively, a water-insoluble dye-starch may be used, followed by detection of the dye bound to the soluble amylose which is the decomposition product of enzymatic reaction. An example of commercially available water-insoluble blue starch polymer is Neoamylase test "Dai-ichi" (produced by Daiichi Pure Chemicals, Co., Ltd.).

### Assaying Process

The analyte antigen, the enzyme-labeled antigen, the antibody and the second antibody are mixed in any order. In general, after the antigen contained in the aqueous liquid sample is mixed with the enzyme-labeled antigen, the antibody (first antibody) is allowed to contact therewith in the solution, followed by adding the second antibody. However, the first antibody may be finally added after the analyte antigen and the enzyme-labeled antibody are mixed with the second antibody in advance. In these cases, the competitive immuoreaction begins to proceed by the addition of the first antibody.

After the first antibody is added to the analyte antigen, the enzyme-labeled antigen and the second antibody may be added successively. In this case, so-called sequential immunological reaction occurs where the first antibody which has not bound to the analyte antigen binds with the enzyme-labeled antigen.

Alternatively, the first antibody is contacted to the enzyme-labeled antigen at first, and then the analyte antigen and the second antibody may be added thereto. In this case, the enzyme-labeled antigen will be liberated from the complex of the antibody and the enzyme-labeled antigen formed in advance, in proportion to the quantity of the analyte antigen added (so-called substitution-release reaction).

In any of the above immunoreaction, it is preferred that the temperature of the solution ranges from 20°C to 45°C, and the pH value of the solution ranges from about 4.0 to about 8.5. A buffer such as phosphate buffer or acetate buffer may be used to maintain the pH value of the solution at the constant level. The time for allowing the antibody to contact with the enzyme-labeled antigen may be determined to ensure completion of the reaction, for example, ranging from 20 to 30 minutes when the temperature of the solution is 37°C. The time for the following reaction with the second antibody may also be determined to ensure completion of the reaction, for example, ranging from 5 to 10 minutes when the temperature of the solution is 37°C.

After a series of immunoreactions described above, a substrate for the enzyme is added to the solution, and the enzymatic activity of the labeling enzyme is measured. The presence of an analyte antigen in the sample can be detected as the increase in enzymatic activity (more precisely, the recovery of the enzymatic activity suppressed). By preparing a calibration curve drawn by using solutions each containing a known quantity of the analyte antigen in advance, the analyte antigen contained in the sample can be quantitatively analyzed.

In an alternative embodiment, only a series of immunoreactions are performed in a solution system and then, the enzymatic activity of the reaction mixture may be analyzed using a dry system. In detail, a dry analysis element having a substrate layer containing the substrate for the labeling enzyme is prepared, and the reaction mixture after the completion of the immunoreactions is applied or spotted on the dry analysis element to measure the enzymatic activity. Such a dry analysis element has the layer structure similar to that described in Unexamined Japanese Patent Publications Nos. 295466/1991, 128655/1992 and the like.

For example, when the labeling enzyme is α-amylase, a substrate layer containing carboxymethylated starch as an insoluble substrate is arranged as the uppermost layer of the dry analysis element. In a reagent layer disposed below the substrate layer, a fragmenting or digesting enzyme (e.g., glucoamylase) for further decomposing an oligomer, which is a decomposition product of the enzymatic reaction of carboxymethylated starch, into the corresponding monomer (glucose) is incorporated. The glucose can optically be detected by a detection reagent composition contained also in the reagent layer. As such a detection reagent composition, a combination of glucose oxidase, peroxidase and leuco dye may be employed.

### Example 1

### Preparation of Phenobarbital-Thiol Compound

100 mg of phenobarbital-valeric acid was dissolved in 1.6 mL of DMF. 34.6 mg of N-hydroxysuccinimide and 57.6 mg of a water-soluble carbodiimide were added thereto and the mixture was stirred at 4°C for 20 hours to prepare an activated phenobarbital. The mixture was then centrifuged to collect the supernatant. To 123 µL of 50 mg/mL mercaptoethylamine hydrochloride solution in DMF was added 201 µL of 50 mg/mL tributylamine solution in DMF. Then, 500 µL of the activated phenobarbital solution (supernatant) prepared as above was added thereto and the mixture was stirred at room temperature for 2.5 hours to prepare a phenobarbital-thiol compound.

### Example 2

### Preparation of GMB Amylase

To 500 µL of a 10 mg/mL solution of *Bacillus subtilis* amylase (in 0.1 M glycerophosphate buffer pH 7.0), 50 µL of a 40 mg/mL solution of ortho-phenanthroline in DMF was added, followed by adding 500 µL of a 100 mg/mL solution of soluble starch (0.1 M glycerophosphate, pH 7.0). 50 µL of a 25 mg/mL solution of GMBS (N-(γ-maleimidobutyryloxy)succinimide) in DMF was added thereto and the mixture was stirred at room temperature for 2 hours. The reaction mixture was applied to SEPHADEX G-25 column, which had been preliminarily equilibrated with a 0.1 M glycerophosphate buffer (pH 7.0), and the passing fraction was collected to obtain (N-(γ-maleimidobutyryloxy)amidated amylase (GMB amylase).

### Example 3

### Preparation of α-Amylase/Phenobarbital Bound

To 600 µL of GMB amylase fraction prepared in Example 2 was added 70 µL of phenobarbital-thiol compound solution prepared in Example 1. The resulting mixture was incubated at 4°C for 20 hours. The reaction mixture was applied to SEPHADEX G-25 column, which had been preliminarily equilibrated with a 0.1 M glycerophosphate buffer (pH 7.0), and the passing fraction was collected to obtain an α-amylase/phenobarbital bound.

### Example 4

### Preparation of Immunoassay Element for α-Amylase

On a colorless and transparent polyethylene terephthalate (PET) sheet (support) coated with a gelatin undercoating layer and having a thickness of 180 µm, coated was a reagent solution containing a cross-linking reagent, followed by drying, to form a reagent layer so that respective components had the coverage as set forth below.

| | |
|---|---|
| Alkaline-treated Gelatin | 14.5 g/m² |
| Nonylphenoxypolyethoxyethanol (Containing 9 to 10 oxyethylene units on average) | 0.2 g/m² |
| Glucose Oxidase | 5,000 U/m² |
| Peroxidase | 15,000 U/m² |
| Glucoamylase | 5,000 U/m² |
| 2-(4-Hydroxy-3,5-dimethoxyphenyl)-4-[4(dimethylamino)phenyl]-5-phenetylimidazole (Leuco Dye) acetate | 0.38 g/m² |
| Bis[(vinylsulfonylmethylcarbonyl)amino]methane | 0.1 g/m² |

Over the resulting reagent layer, an adhesive layer was formed by applying the following reagents, followed by drying, to have the following coverage.

| | |
|---|---|
| Alkaline-treated Gelatin | 14.5 g/m² |
| Bis[(vinylsulfonylmethylcarbonyl)amino]methane | 0.1 g/m² |

Then, an aqueous solution containing the following reagents was coated over the surface of the resulting adhesive layer to have the following coverage. The gelatin layer was swollen and a tricot knitted cloth made by knitting PET spun yarn of 36 gauge corresponding to 50 deniers and having a thickness of about 250 µm was laminated thereon under a uniform and light pressure, whereby a porous spreading layer was formed.

| | |
|---|---|
| Nonylphenoxypolyethoxyethanol (containing 9 to 10 oxyethylene units on average) | 0.15 g/m² |
| Bis [(vinylsulfonylmethylcarbonyl)amino]methane | 0.4 g/m² |

Thereafter, a substrate layer was formed by applying a substrate, followed by drying, to have the following coverage, whereby a multi-layered analysis element for the quantitative analysis of α-amylase was prepared.

| | |
|---|---|
| Carboxymethylated starch | 5 g/m² |
| Nonylphenoxypolyethoxyethanol (containing 9 to 10 oxyethylene units on average) | 0.2 g/m² |

The thus prepared analysis element was cut into a rectangular tip of 15 mm x 15 mm, and the tip was placed in a slide frame described in Unexamined Japanese Patent Publication No. 63452/1982 to prepare a multi-layered dry slide for the analysis of α-amylase.

### Example 5

A reaction mixture containing 0.02 mg/mL α-amylase/phenobarbital bound obtained in Example 3, 0.05 mg/mL anti-phenobarbital antibody (monoclonal) as a first antibody, 1 mg/mL goat anti-mouse IgG (Fc) antibody (product of Jackson Immunoresearch) and 50 mM MES buffer (pH 6.5) was prepared and incubated at 37°C for 20 minutes to cause an immunoreaction. After completion of the immunoreaction, 10 µL of the resulting solution was spotted on the α-amylase analysis slide prepared in Example 4. The slide was maintained at 37°C, and the optical density of the reflected light having a central wavelength of 650 nm was measured from the PET support side. The difference in optical density (ΔOD₆₋₄) between the optical densities of the reflected lights measured respectively after the lapse of 4 minutes and 6 minutes from spotting was determined, and the value was assigned to enzymatic activity.

As Comparative Example as a reference, an operation similar to the Example was performed except that the second antibody was not added to the reaction mixture. AS Control, the enzymatic activity was measured in a similar manner to the Example except that neither the first nor the second antibody was added to the reaction mixture. The enzyme inhibition rate was determined in the Example and Comparative Example.

As shown in Table 1, while the inhibition rate for the labeling enzyme α-amylase with using the first antibody alone was 68%, the inhibition rate was remarkably improved to 94% by adding the second antibody.

**Table 1**

| | Antibody | Second Antibody | Amylase Activity | Inhibition Rate |
|---|---|---|---|---|
| Control | - | - | 100% | - |
| Comparative Example | + | - | 32% | 68% |
| Example 5 | + | + | 6% | 94% |

### Example 6

50 µL of 50 mM MES buffer solution (pH 6.5) containing a known amount of phenobarbital was added to 50 µL of the α-amylase/phenobarbital bound (0.04 mg/mL) obtained in Example 3, and then, 50 µL of mouse anti-phenobarbital antibody (0.1 mg/mL; monoclonal) was further added thereto, followed by incubation at 37°C for 20 minutes. Then, 10 µL of the resulting solution was spotted on the α-amylase analysis slide prepared in Example 4. The slide was maintained at 37°C and the optical density of the reflected light having a wavelength of 650 nm was measured from the PET support side. The difference in optical density (ΔOD₆₋₄) between the optical densities of the reflected lights measured respectively after the lapse of 4 minutes and 6 minutes after spotting was determined. A calibration curve of Comparative Example was prepared based on the result of determination.

As the Example 6, a similar operation was performed except that a goat anti-mouse IgG (Fc) antibody (product of Jackson Immunoresearch) was added into the α-amylase/phenobarbital bound (0.04 mg/mL) solution of the above-described Comparative Example.

Each of the calibration curves is shown in FIG. 1. As shown in FIG. 1, background value at low antigen concentration side was remarkably lowered by the addition of the second antibody (anti-Fc antibody). In addition, at high antigen concentration side, recovery of the labeling enzyme activity was similar to that in Comparative Example (anti-Fc antibody (-)) wherein the second antibody was not used. Thus, in the present example wherein the second antibody was used, the S/N ratio was remarkably improved (FIG. 1; -○-). Moreover, the addition of the second antibody neither affected the detection limit nor lowered the sensitivity.

### Example 7

Similar to Example 4, a multi-layered analysis element having a tricot knitted cloth layer was prepared. On the tricot knitted cloth layer, which served both as a substrate layer and a spreading layer, coated was an aqueous solution of a mouse anti-phenobarbital antibody (monoclonal) to have a coverage of 51.6 mg/m², followed by drying, to prepare a multi-layered immunoassay slide 2 containing the first antibody for the analysis of phenobarbital.

### Example 8

50 µL of 50 mM MES buffer solution (pH 6.5) containing a known quantity of phenobarbital was added to 50 µL of the α-amylase/phenobarbital bound solution (0.04 mg/mL) obtained in Example 3, and then, 50 µL of a goat anti-mouse IgG (Fc) antibody solution (1 mg/mL, product of Jackson Immunoresearch) was further added thereto, followed by incubation at 37°C for 20 minutes.

Then, 10 µL of the resulting solution was spotted on the antibody-containing slide 2 prepared in Example 7. The slide was maintained at 37°C and the optical density of the reflected light having a wavelength of 650 nm was measured from the PET support side. The difference in optical density (ΔOD₆₋₄) between the optical densities of the reflected lights measured respectively after the lapse of 4 minutes and 6 minutes after spotting was determined. A calibration curve was prepared based on the result of determination.

As Comparative Example, an operation similar to the Example was performed except that a goat anti-mouse IgG (Fc) antibody was not added to the MES buffer solution. A calibration curve was prepared based on the result of determination.

Each of the calibration curves obtained is shown in FIG. 2. As shown in FIG. 2, even when the dry slide containing the first antibody was used, improvement of the S/N ratio was observed (FIG. 2, -○-) by adding the second antibody (anti-Fc antibody). Moreover, the addition of the second antibody neither affected the detection limit nor lowered the sensitivity.

### Example 9

### Preparation of Phenytoin-Thiol Compound

102 mg of phenytoin-valeric acid was dissolved in 1.6 mL of DMF. 34.6 mg of N-hydroxysuccinimide and 57.6 mg of a water-soluble carbodiimide were added thereto and the mixture was stirred at 4°C for 20 hours to prepare an activated phenytoin. The mixture was then centrifuged to collect the supernatant. To 123 µL of 50 mg/mL mercaptoethylamine hydrochloride solution in DMF was added 201 µL of 50 mg/mL tributylamine solution in DMF. Then, 500 µL of the activated phenytoin solution (supernatant) prepared as above was added thereto and the mixture was stirred at room temperature for 2.5 hours to prepare a phenytoin-thiol compound.

### Example 10

### Preparation of GMB Amylase

To 500 µL of 10 mg/mL *Bacillus subtilis* amylase solution in 0.1 M glycerophospate (pH 7.0), 50 µL of 40 mg/mL ortho-phenanthroline solution in DMF was added, followed by adding 500 µL of a 100 mg/mL solution of soluble starch (in 0.1 M glycerophosphate, pH 7.0). 50 µL of a 25 mg/mL solution of GMBS (N-(γ-maleimidobutyryloxy)succinimide) in DMF was added thereto and the mixture was stirred at room temperature for 2 hours. The reaction mixture was subjected to gel filtration by SEPHADEX G-25 column, which had been preliminarily equilibrated with a 0.1 M glycerophosphate buffer (pH 7.0), and the passing fraction was collected to obtain (N-(γ-maleimidobutyryloxy)amidated amylase (GMB amylase).

### Example 11

### Preparation of α-Amylase/Phenytoin Bound

70 µL of phenytoin-thiol compound solution obtained in Example 9 was added to 600 µL of GMB amylase solution prepared in Example 10. The resulting mixture was incubated at 4°C for 20 hours. The reaction mixture was applied to SEPHADEX G-25 column, which had been preliminarily equilibrated with a 0.1 M glycerophosphate buffer (pH 7.0), and the passing fraction was collected to obtain an α-amylase/phenytoin bound.

### Example 12

50 µL of 50 mM MES buffer solution (pH 6.5) containing a known quantity of phenytoin was added to 50 µL of a solution containing the α-amylase/phenytoin bound (0.04 mg/mL) obtained in Example 11 and a goat anti-mouse IgG (Fc) antibody (1 mg/mL; product of Jackson immunoresearch) as the second antibody. Then, 50 µL of a 0.011 mg/mL solution of monoclonal mouse anti-phenytoin antibody as the first antibody was added thereto, followed by incubation at 37°C for 20 minutes. Thereafter, 10 µL of the resulting solution was spotted on the α-amylase analysis slide prepared in Example 4. The slide was maintained at 37°C and the optical density of the reflected light having a wavelength of 650 nm was measured from the PET support side. The difference in optical density (ΔOD₆₋₄) of the reflected lights measured respectively after the lapse of 4 minutes and 6 minutes was determined. A calibration curve was prepared based on the result of determination.

As Comparative Example, a similar operation was performed except that the goat anti-mouse IgG (Fc) antibody as the second antibody was not used and the concentration of the monoclonal mouse anti-phenytoin antibody added as the first antibody was changed to 0.022 mg/mL which was twice that in the above-described example. A calibration curve was prepared based on the result of determination.

Each of the calibration curves is shown in FIG. 3. As shown in FIG. 3, the value (background value) at low antigen concentration side was high and the S/N ratio was about 2 in Comparative Example wherein the second antibody was not used (FIG. 3, -●-).

On the contrary, when the second antibody was used, a remarkable suppression of the enzyme activity was observed and the background value was sufficiently lowered at low antigen concentration side. The S/N ratio was increased to about 10 (FIG. 3, -○-). In addition, since the quantity of the first antibody used in Example was reduced to one half of that in Comparative Example, the detection limit shifted from about 0.08 µg/mL in Comparative Example to about 0.02 µg/mL in the present example which is about one quarter of the former. Namely, in the present example, the sensitivity was about 4 times as high as that in Comparative Example, and a remarkable improvement of the S/N ratio was observed as compared with that in Comparative Example.

As has been described in detail hereinbefore, in the present invention, a ligand (analyte antigen) is subjected to an immunoreaction between the ligand, an enzyme-labeled ligand, a first antibody against the ligand, and a second antibody against the first antibody, and then the change in enzymatic activity of the labeling enzyme in the reaction mixture was determined. By using the additional second antibody, a sufficient S/N ratio can be ensured together with a low background value, though such a sufficient S/N ratio is hardly obtainable when the first antibody alone is added as antibody. In addition, even when the quantity of the antibody relative to the antigen is reduced in order to increase the sensitivity, the S/N ratio is not lowered, and the observed S/N ratio is rather higher than before reducing the quantity of the antibody.

## Claims

1. A homogeneous enzyme immunoassay process for quantitatively analyzing a ligand, comprising of:
reacting the ligand with an enzyme-labeled ligand, an antibody against the ligand, and a second antibody against said antibody; and then
measuring a change in enzymatic activity of a labeling enzyme of said enzyme-labeled ligand to determine the quantity of said ligand.

2. The process according to claim 1, wherein the enzymatic activity of the labeling enzyme in the reaction mixture is determined from a water-insoluble substrate.

3. The process according to claim 2, wherein the enzymatic activity of the labeling enzyme in the reaction mixture is determined by a dry analysis element equipped with a substrate layer containing said water-insoluble substrate.

## Patentansprüche

1. Homogenes Enzym-Immunoassay-Verfahren zum quantitativen Analysieren eines Liganden, umfassend:
Umsetzen des Liganden mit einem Enzym-markierten Liganden, einem Antikörper gegen den Liganden und einem zweiten Antikörper gegen den genannten Antikörper und dann
Messen der Änderung der enzymatischen Aktivität eines Markierungsenzyms des Enzym-markierten Liganden, um die Menge des Liganden zu bestimmen.

2. Verfahren nach Anspruch 1, wobei die enzymatische Aktivität des Markierungsenzyms im Reaktionsgemisch von einem wasserunlöslichen Substrat bestimmt wird.

3. Verfahren nach Anspruch 2, wobei die enzymatische Aktivität des Markierungsenzyms im Reaktionsgemisch durch ein Trockenanalyseelement, das mit einer Substratschicht ausgestattet ist, welche das wasserunlösliche Substrat enthält, bestimmt wird.

## Revendications

1. Procédé d'immunoanalyse enzymatique homogène pour analyser quantitativement un ligand, comprenant :
la réaction du ligand avec un ligand marqué avec une enzyme, et un anticorps contre le ligand, et un second anticorps contre ledit anticorps ; puis
la mesure d'un changement d'activité enzymatique d'une enzyme de marquage dudit ligand marqué avec une enzyme pour déterminer la quantité dudit ligand.

2. Procédé selon la revendication 1, où l'activité enzymatique de l'enzyme de marquage dans le mélange réactionnel est déterminée à partir d'un substrat insoluble dans l'eau.

3. Procédé selon la revendication 2, où l'activité enzymatique de l'enzyme de marquage dans le mélange réactionnel est déterminée par un élément d'analyse sec équipé d'une couche de substrat contenant ledit substrat insoluble dans l'eau.
